# EUROPEAN PATENT APPLICATION

(11) **EP 2 033 955 A1**
(43) Date of publication of application: **11.03.2009**
(21) Application number: 08015666.4
(22) Date of filing: 05.09.2008
(51) Int. Cl.: C07D 231/38, A61K 31/415, A61K 31/4155, A61K 31/497, A61P 29/00

(54) **Urea derivatives of 1H-pyrazol-4-carboxylic acid with neutrophil chemotaxis inhibiting activity**

(30) Priority: 06.09.2007 IT Mi20071731
(71) Applicant: Universita' Degli Studi Di Genova, 16145 Genova (IT)
(72) Inventor: Bruno, Olga, 16132 Genova (IT); Bondavalli, Francesco, 16132 Genova (IT); Brullo, Chiara, 16132 Genova (IT); Schenone, Silvia, 16132 Genova (IT)
(74) Representative: Ferreccio, Rinaldo

(57) **Abstract**

Compounds of formula (I), 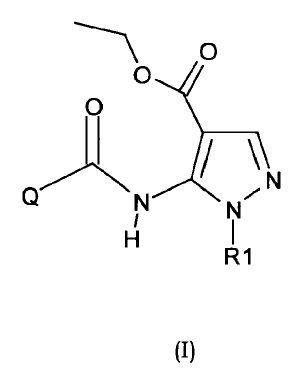 wherein R₁ = H, optionally substituted C₁-C₈ alkyl, -CH₂-CH(R₃)OR₂, where R₂ = H or C₁-C₃ alkyl and R₃ = optionally substituted C₁-C₈ alkyl; Q = NHR₄ or a saturated heterocyclic 3-7 membered ring containing nitrogen, where R₄ = optionally substituted C₃-C₈ alkyl, optionally substituted aralkyl, optionally substituted aryl; and enantiomers, diastereoisomers and pharmaceutically acceptable salts thereof; it is also described a process for preparing such compounds, the use of such compounds as a medicament, in particular in the treatment of inflammatory diseases of autoimmune origin, as well as pharmaceutical compositions that comprise such compounds.

## Description

### Field of Application

The present invention concerns the technical sector of the pharmaceutical industry.

In particular, the invention refers to new urea derivatives of 1H-pyrazole-4-carboxylic acid which are neutrophil chemotaxis inhibitors and thususeful in the treatment and/or prevention of inflammatory diseases.

### Prior Art

As known, inflammation constitutes the first response of the immune system to infection or irritation and generally results in connective tissue scarring¹. Moreover, should the action of the infectious or irritating agent go on or should the control of cellular recruitment be lost, autoimmune inflammatory disorders, either acute or chronic (such as, for example, asthma, rheumatoid arthritis, multiple sclerosis and inflammatory bowel disease), will ensue.

Neutrophils are the main cells infiltrated in the course of acute inflammation. Their activation comprises different events: margination (rolling and adhesion onto blood vessels), diapedesis (transmigration across the endothelial barrier), chemotaxis (migration to the inflammation site), phagocytosis of foreign particles and production of reactive free radicals^{2,3}.

Chemotaxis is a multistep process⁴ induced by different chemoattractants, such as fMLP (N-formyl-methionyl-leucylphenylalanine),^{5,6} PAF (platelet activation factor),⁷ LTB4 (leukotriene B4),⁸ C5a anaphylotoxin⁹ and different cytokines such as CC chemokine MIP 1β or CXC chemokine IL8 (Interleukin 8 also named CXCL8).¹⁰

Both the responses to IL8 and those to fMLP are induced by the activation of specific G-protein-coupled receptors, expressed on target cells.¹¹⁻¹⁶

Downstream from the trimeric G proteins a number of intracellular signalling systems are activated, including (a) the stimulation of phospholipase C, followed by the activation of protein kinase C and the increase of cytosolic calcium, (b) the activation of enzyme PI3K (phosphoinositide 3-kinase), resulting in an increase of PIP3 (phosphatidylinositol 3,4,5-trisphosphate) and PIP2 (phosphatidylinositol 3,4-bisphosphate), and (c) activation of small GTP-binding proteins of the Rho family ("Ras homology gene") and of the MAPK cascade (mitogen-activated protein kinase), as well as activation of protein kinases and protein phosphatases.¹⁷

This complex system results in the rapid development of ruffles on the cell body and in the formation of polymerised F-actin in the ruffles. The cells then develop a polarized form with the formation of a contracted tail in the rear, and F-actin-rich ruffles at the front.¹⁸⁻²⁰ The newly formed filaments of polymerised actin are enriched in the leading edge of a migrating cell, ^{21,22} and the increase in filamentous actin causes the formation of pseudopods.²³⁻²⁵ The block of actin polymerisation abolishes chemotaxis.^{26,27}

The F-actin polymerisation mechanism has not yet been fully understood;³⁰ it depends on the activation of PI3K, in particular on the phosphorylation of PI3Kγ^{31,32} and PI3Kδ³³ isoforms and on the production of PIP3; moreover, the activation of the protein kinase B (Akt/PKB) constitutes a major downstream regulator of PI3K-dependent chemotaxis.^{4,34,35}

Two distinct pathways of actin polymerisation have recently been reported. The PI3K-dependent signaling depends on PKC-ζ and Akt; the PI3K-independent signaling depends instead on ROCK-kinases (RhoA/Rho-kinase), tyrosine kinases of the Src family and NADPH, and is modulated by cAMP. The different chemotactic receptors use these distinct pathways.^{36,37}

The interest of many academic and pharmaceutical industry researchers has recently been directed to new molecules that are capable of interfering with the neutrophil upregulation.

A potent non-peptide IL8 inhibitor was first synthesised, which binds to the CXCR2 receptor: SB 225002 [N-(2-hydroxy-4-nitrophenyl)-N'-(2-bromophenyl)urea]³⁸. This compound was followed by many molecules, which act at CXC receptors^{39,40}, some of which have shown interesting pharmacological activities in vivo (for example N-(3-aminosulfonyl)-4-chloro-2-hydroxyphenyl)-N'-(2,3 dichlorophenyl)urea.

The inventors of the present application have been working for many years on the synthesis of new heterocyclic derivatives as potential anti-inflammatory agents and their interest has recently been focused on the identification of new molecules capable of impacting neutrophil activation and migration⁴¹.

In particular, a series of N-pyrazolyl-N'-alkyl/benzyl/phenylureas have been synthesised, which constitute a new class of potent inhibitors of the neutrophil chemotaxis induced by Interleukin 8 (Bruno O. et al., Journal of Medicinal Chemistry, 2007, 50 (15) 3618-3626 *Synthesis and biological evaluation of N-Pyrazolyl-N'-alkyl*/*benzyl*/*phenylureas: a new class of potent inhibitors of Interleukin 8-induced neutrophil chemotaxis*).

Such compounds exhibit the following general formula 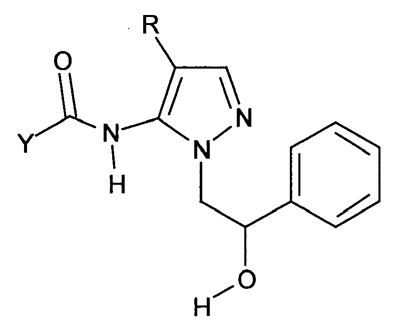 wherein R = COOC₂H₅ or CN or H and Y is for example optionally substituted -NH-phenyl, optionally substituted -NH-benzyl, -NH-isopropyl, 1-cyclopropylamino, 1-pyrrolidinyl.

It has been shown that the above-mentioned compounds are capable of inhibiting the chemotaxis induced by IL8 and, for the more active compounds, an IC50 in the range of 0.01 µM has been determined. The above compounds have shown instead to be inactive in relation to the chemotaxis induced by fMLP.

Patent application EP 0 810 217 describes pyrazole derivatives for which an activity has been reported as smooth muscle cells growth inhibitors. The compounds N-phenyl-N'-(4-ethoxycarbonyl)-1-methylpyrazol-5-yl)urea and N-phenyl-N'-(4-ethoxycarbonyl)-1H-pyrazol-5-yl)urea are specifically described.

Macon, Z. et al., Acta Poloniae Pharmaceutica (1985), 42(6), 516-20, describe compounds of the following formula 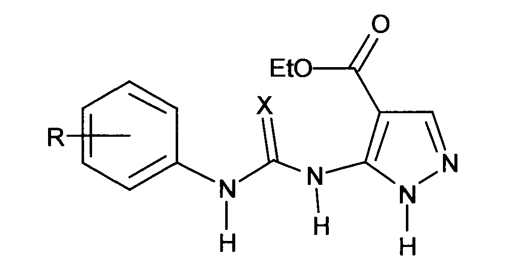 wherein X = O, S and R = H, 3-Cl, 4-Cl, as side products isolated in the synthesis of pyrazole derivatives and active in preventing experimental oedema.

The following compound is specifically described:
N-(4-chlorophenyl)-N'-(4-ethoxycarbonyl)-1H-pyrazol-5-yl)urea.

From patent application JP 56156264, the following compounds endowed with herbicidal activity are known:
N-(3-chlorophenyl)-N'-(4-ethoxycarbonyl)-1H-pyrazol-5-yl)urea.
N-(2-chlorophenyl)-N'-(4-ethoxycarbonyl)-1H-pyrazol-5-yl)urea.

### Summary of the Invention

The problem at the basis of the present invention has been to provide new compounds endowed with a powerful inhibitory activity in relation to the chemotaxis induced by interleukin 8, which may be used in the treatment of inflammatory diseases and, in particular, of autoimmune inflammatory diseases, either acute or chronic.

Such a problem was solved by compounds of the following formula (I), 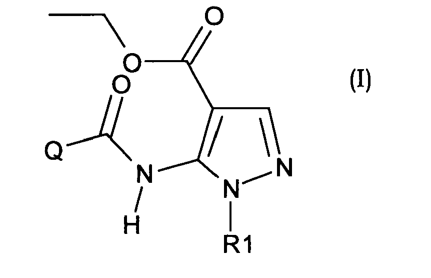 wherein:
R₁ = H, optionally substituted C₁-C₈ alkyl, -CH₂-CH(R₃)OR₂, wherein R₂ = H or C₁-C₃ alkyl and R₃ = optionally substituted C₁-C₈ alkyl;
Q = NHR₄, or a saturated heterocyclic 3-7 membered ring containing nitrogen
where R₄ = optionally substituted C₃-C₈ alkyl, optionally substituted aralkyl, optionally substituted aryl;
provided that, when R₁ = H or CH₃, Q is different from anilino and chloroanilino,
and by enantiomers, diastereoisomers and pharmaceutically acceptable salts thereof.

The above-mentioned optionally substituted alkyl, aryl, aralkyl bear one or more substituents chosen from the group comprising C₁-C₃ alkyl, halogen, C₁-C₃ alkoxy, hydroxy, SH, C₁-C₃ alkylthio, nitro and haloalkyl.

C₁-C₈ alkyl indicates monovalent alkyl groups that have from one to eight carbon atoms; by way of example groups such as methyl, ethyl, n-propyl, isopropyl, n-butyl, isobutyl, terbutyl and the like are mentioned.

"Aryl" indicates aromatic carbocyclic groups having from 6 to 14 carbon atoms, with a single ring (e.g. phenyl) or with multiple condensed rings (e.g. naphthyl). Included among preferred aryls are phenyl, biphenyl, naphthyl, phenanthrenyl.

"Aralkyl" indicates an aryl as defined above bonded to an alkyl group having from 1 to 3 carbon atoms. Included among preferred aralkyls are benzyl, phenylethyl, naphthylmethyl, naphthylethyl.

Preferred are the compounds of formula (I) wherein:
R₁ = C₁-C₅ alkyl, -CH₂-CH(R₃)OR₂, where R₃ = C₁-C₃ alkyl and R₂ is defined as above;
Q = -NH-CH(CH₃)₂, -NH-CH₂-C₆H₅, -NH-C₆H₄X, N-benzylpiperazino, cyclopropylamino, pyrrolidino, piperidino, morpholino, N-methylpiperazino or α-naphthylamino, where X = halogen.

More preferred are the compounds wherein:
Q = -NH-CH(CH₃)₂, -NH-CH₂-C₆H₅, -NH-C₆H₄X, N-benzylpiperazino, α-naphthylamino, where X = halogen.

Particularly preferred are the compounds of formula (I) wherein:
R₁ = -CH₂-CH(OH)CH₃, -CH₂-CH(OH)C₂H₅, -CH₂-CH(OH)C₃H₇ and
Q = -NH-CH(CH₃)₂, -NH-CH₂-C₆H₅, N-benzylpiperazino, α-naphthylamino, o-fluoroanilino and m-fluoroanilino.

The more preferred compounds according to the present invention are those bearing the substituents indicated in the following table.

| R₁ | Q |
|---|---|
| CH₂-CHOH-CH₃ | isopropylamino |
| CH₂-CHOH-CH₃ | benzylamino |
| CH₂-CHOH-CH₃ | N-benzylpiperazino |
| CH₂-CHOH-CH₃ | o-F-anilino |
| CH₂-CHOH-CH₃ | m-F-anilino |
| CH₂-CHOH-CH₃ | α-naphthylamino |
| CH₂-CHOH-C₂H₅ | isopropylamino |
| CH₂-CHOH-C₂H₅ | benzylamino |
| CH₂-CHOH-C₂H₅ | N-benzylpiperazino |
| CH₂-CHOH-C₂H₅ | o-F-anilino |
| CH₂-CHOH-C₃H₇ | benzylamino |
| CH₂-CHOH-C₃H₇ | isopropylamino |

The present invention also concerns a process for the production of the above-mentioned compounds of formula (I) from an intermediate compound of formula (II) 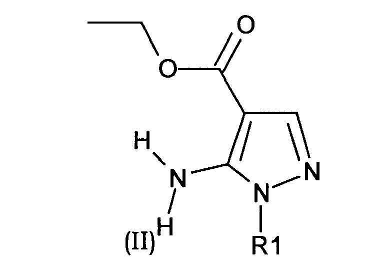 wherein R₁ has the same meanings assigned for the compounds of formula (I). According to such process, the 5-aminopyrazole compound of formula (II) is converted into a compound of formula (I) according to the invention by reacting it with:
A) COCl₂ and subsequently with an amine of formula QH, where Q has the meanings indicated above, or with
B) an isocyanate of formula QNCO, where Q represents an optionally substituted aryl.

The reaction with phosgene is generally conducted in anhydrous tetrahydrofuran; the reaction with the isocyanate is generally conducted in anhydrous toluene at reflux.

The intermediate 5-aminopyrazole compound (II) is in turn prepared by means of condensation of ethyl(ethoxymethylene)cyanoacetate with hydrazine derivatives of formula R₁NHNH₂ (III), wherein R₁ has the above meanings, according to the following scheme

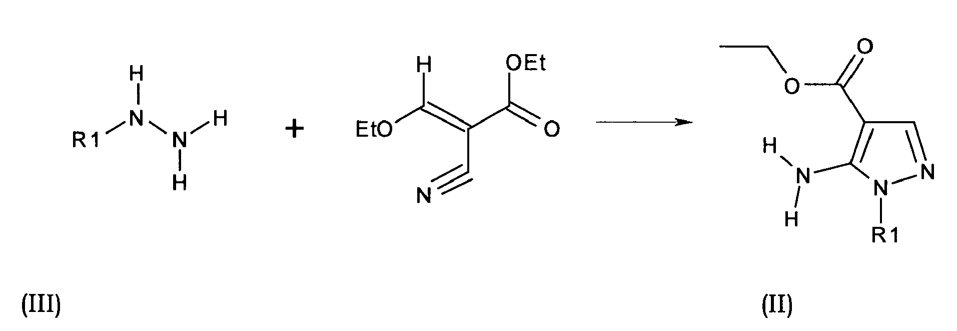

The hydrazines R₁NHNH₂ are commercially available or, in the case in which R₁ is -CH₂-CH(R₃)OH or -CH₂-CH(C₆H₅)OH, they are prepared through reaction between hydrazine hydrate and an epoxy compound of formula W-(CH-CH₂)O, wherein W is = R₃ or C₆H₅, according to the following scheme:

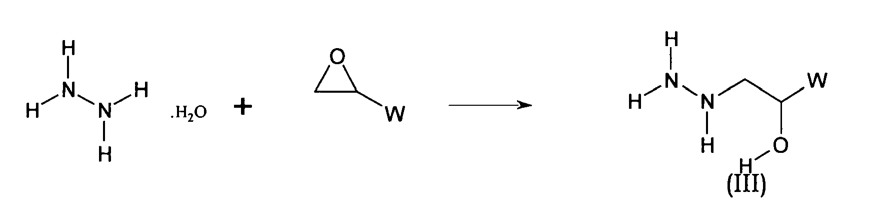

The reaction is generally run at a temperature of 50-100°C.

The present invention further concerns compounds of formula (I) 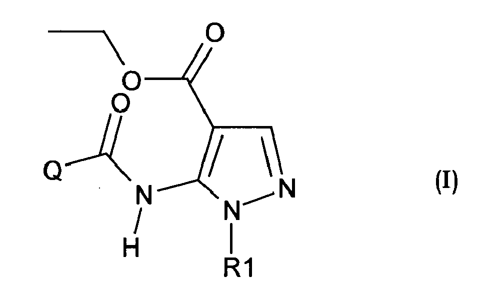 wherein R₁ = H, optionally substituted C₁-C₈ alkyl, -CH₂-CH(R₃)OR₂,
where R₂ = H or C₁-C₃ alkyl and R₃ = optionally substituted C₁-C₈ alkyl;
Q = NHR₄, N-benzylpiperazino, cyclopropylamino, pyrrolidino, piperidino, morpholino, N-methylpiperazino,
where R₄ = optionally substituted C₃-C₈ alkyl, optionally substituted aralkyl, optionally substituted aryl, and their enantiomers, diastereoisomers and pharmaceutically acceptable salts for use as a medicament with anti-inflammatory activity.

The above-mentioned optionally substituted alkyl, aryl, aralkyl bear one or more substituents chosen from the group comprising C₁-C₃ alkyl, halogen, C₁-C₃ alkoxy, hydroxy, SH, C₁-C₃ alkylthio, nitro and haloalkyl.

Preferred are the compounds of formula (I) wherein:
R₁ = C₁-C₅ alkyl, -CH₂-CH(R₃)OR₂, where R₃ = C₁-C₃ alkyl and R₂ is defined as above;
Q = -NH-CH(CH₃)₂, -NH-CH₂-C₆H₅, -NH-C₆H₄X, N-benzylpiperazino, α-naphthylamino, where X = halogen.

Particularly preferred are the compounds of formula (I) wherein:
R₁ = -CH₂-CH(OH)CH₃, -CH₂-CH(OH)C₂H₅, -CH₂-CH(OH)C₃H₇ and
Q = -NH-CH(CH₃)₂, -NH-CH₂-C₆H₅, -NH-C₆H₄X, N-benzylpiperazino, cyclopropylamino, pyrrolidino, piperidino, morpholino, N-methylpiperazino or α-naphthylamino, where X = halogen.

More preferred are the compounds wherein:
Q = -NH-CH(CH₃)₂, -NH-CH₂-C₆H₅, N-benzylpiperazino, α-naphthylamino, o-fluoroanilino and m-fluoroanilino.

The invention further concerns the use of such compounds of formula (I) proposed for use as a medicament in the production of a medicament for the treatment of inflammatory pathologies of autoimmune nature.

Examples of inflammatory pathologies in the treatment of which the compounds according to the present invention may find use are asthma, multiple sclerosis, inflammatory bowel disease, ulcerative colitis, dermatitis, in particular atopic dermatitis and contact dermatitis, psoriasis, inflammation following organ transplantation, acute respiratory failure (ARDS - "Acute Respiratory Distress Syndrome"), cystic fibrosis, chronic obstructive lung disease, glomerulonephritis.

The compounds according to the present invention are also indicated for the prevention and treatment of damages fromischemia -reperfusion.

The present invention further refers to a pharmaceutical composition comprising a compound of formula (I) as defined above or an enantiomer or diastereoisomer or pharmaceutically acceptable salt thereof and a pharmaceutically acceptable carrier.

Examples of pharmaceutically acceptable salts are those formed with organic acids such as oxalic, tartaric, maleic, succinic and citric and with inorganic acids such as nitric, hydrochloric, sulphuric and phosphoric.

The compounds according to the invention that have one or more asymmetric carbon atoms may exist as pure enantiomers, as pure diastereoisomers, as racemic mixtures of enantiomers, racemates and mixtures of racemates.

The compounds and compositions according to the invention may be administered with any available and efficient delivery system, comprising, but not limited to, oral, buccal, parenteral, inhalatory routes, topical application, by injection, by transdermic or rectal route (for ex. by means of suppositories) in dosage unit formulations containing conventional, pharmaceutically acceptable and non-toxic carriers, adjuvants and vehicles. The administration by parenteral route comprises subcutaneous, intravenous, intramuscular, intrasternal injection or infusion techniques.

The solid dosage forms for the administration by oral route comprise, for example, capsules, tablets, powders, granules and gels. In such solid dosage forms, the active compound may be mixed with at least one inert diluent such as, for example, sucrose, lactose or starch. These dosage forms normally also comprise additional substances different from the inert diluents, such as, for example, lubricating agents like magnesium stearate.

The injectable preparations, for example aqueous or oily sterile injectable solutions or suspensions, may be formulated according to the known technique and by optionally using appropriate dispersing, wetting and/or suspending agents.

The pharmaceutical preparations according to the present invention may be produced by using conventional pharmaceutical techniques, as described in the various pharmacopoeias or handbooks of the field such as, for example, "Remington's Pharmaceutical Sciences Handbook", Mack Publishing, New York, 18th Ed., 1990.

The average daily dosage of the compounds according to the present invention depends on many factors, such as, for example, the seriousness of the disease and the conditions of the patient (age, weight, sex): The dose may generally vary from 1 mg to 1500 mg per day of compound according to the invention, optionally divided into more administrations.

### Detailed Description

The present invention will be further described by referring to a few examples of preparation of compounds according to the invention and of evaluation of their activity as inhibitors of the neutrophil chemotaxis.

Among the compounds according to the present invention, those indicated in the following table are mentioned in particular.

| **Compound** | **R₁** | **Q** |
|---|---|---|
| **Ia** | CH₂-CHOH-C₆H₅ | α-naphthylamino |
| **Ib** | CH₂-CHOH-CH₃ | isopropylamino |
| **Ic** | CH₂-CHOH-CH₃ | benzylamino |
| **Id** | CH₂-CHOH-CH₃ | N-benzylpiperazino |
| **Ie** | CH₂-CHOH-CH₃ | *o-*F-anilino |
| **If** | CH₂-CHOH-CH₃ | *m*- F-anilino |
| **Ig** | CH₂-CHOH-CH₃ | *p*- F-anilino |
| **Ih** | CH₂-CHOH-CH₃ | α-naphthylamino |
| **Ii** | CH₂-CHOH-C₂H₅ | isopropylamino |
| **Ij** | CH₂-CHOH-C₂H₅ | benzylamino |
| **Ik** | CH₂-CHOH-C₂H₅ | N-benzylpiperazino |
| **Il** | CH₂-CHOH-C₂H₅ | *o*- F-anilino |
| **Im** | CH₂-CHOH-C₂H₅ | *m*- F-anilino |
| **In** | CH₂-CHOH-C₂H₅ | *p*- F-anilino |
| **Io** | CH₂-CHOH-C₂H₅ | α-naphthylamino |
| **Ip** | CH₂-CHOH-C₃H₇ | isopropylamino |
| **Iq** | CH₂-CHOH-C₃H₇ | benzylamino |
| **Ir** | CH₂-CHOH-C₃H₇ | N-benzylpiperazino |
| **Is** | CH₃ | isopropylamino |
| **It** | CH₃ | benzylamino |
| **Iu** | CH₃ | N-benzylpiperazino |

Their preparation is indicated hereinafter.

### EXAMPLE 1

### Synthesis of 1-(2-hydroxy-2-phenylethyl)-5-[3-(1-naphthyl)ureido]-1H-pyrazole-4-carboxylic acid ethyl ester (Ia)

To a suspension of 5-amino-1-(2-hydroxy-2-phenylethyl)-1H-pyrazole-4-carboxylic acid ethyl ester (1.38 g, 5 mmoles) in anhydrous toluene (10 ml), α-naphthylisocyanate (1.02 g, 6 mmoles) was added and the reaction mixture was refluxed for 6 hours. After cooling, the solvent was evaporated under reduced pressure and the crude product was dissolved in CH₂Cl₂ (10 ml), washed with water (2x10 ml) and with 1M HCl, dried (MgSO₄) and concentrated under reduced pressure. The crude product is crystallised by addition of a mixture of diethyl ether/petroleum ether (boiling point 40-60°C) (1:1), yielding a white solid.

Yield 52%, melting point 181-183°C. ¹H-NMR (CDCl₃) δ 1.26 (t, J=7.2, 3H, CH₃), 1.61 (br s, 2H, disappears with D₂O) 4.06-4.28 (m, 4H, CH₂O + CH₂N), 5.93-6.04 (m, 1H, CHOH), 7.14-7.90 (m, 14H, 12HAr + H₃ + 1H which disappears with D₂O). IR (KBr): cm⁻¹ 3447, 3393, 3328 (NH, OH), 1710 (COOEt), 1676 (CONH). Anal. (C₂₅H₂₄N₄O₄) C, H, N. (% calculated / found) C: 67.54 / 67.40; H: 5.45 / 5.74; N: 12.61 / 12.63.

### General procedure for 1-(2-hydroxyalkyl)-5-(3-alkyl/cycloalkylureido)-1H-pyrazole-4-carboxylic acids ethyl esters Ib-d and for 1-methyl-5-(3-alkyl/cycloalkylureido)-1H-pyrazole-4-carboxylic acids ethyl esters Ip-u

To a mixture of the appropriate 5-amino-1-(2-hydroxyalkyl-)-1*H-*pyrazole-4-carboxylic acid ethyl ester (10 mmoles) and anhydrous CH₃COONa (2.0 g, 24 mmoles) in anhydrous THF (30 ml), cooled at 0°C, phosgene (20% in toluene, 8 ml) was added dropwise; successively the reaction mixture was heated at reflux for 4 h. After removal in vacuo of the excess phosgene, the appropriate amine was added (25 mmoles), dissolved in anhydrous THF, dropwise at 0°C; the mixture was stirred at room temperature overnight. After removal of the volatiles in vacuo, the crude product was suspended in water (20 ml), extracted twice with CH₂Cl₂ (20 ml) and dried (MgSO₄). The solvent was evaporated under reduced pressure to yield yellow oils, which were purified by means of chromatography (diethyl ether) on silica gel column (0.06-0.2 mm). The white solids obtained were recrystallised from absolute ethanol.

### EXAMPLE 2

### 1-(2-hydroxypropyl)-5-(3-isopropylureido)-1H-pyrazole-4-carboxylic acid ethyl ester Ib

Yield 58%, melting point 93-94 °C. ¹H-NMR (CDCl₃): δ 1.19 (d, J=6.6, 6H, 2CH₃ isoprop.), 1.30-1.46 (m, 6H, 2CH₃), 3.72-3.90 (m, 1H, CHNH), 3.99-4.13 (m, 2H, CH₂N), 4.29 (q, J=7.2, 2H, CH₂O), 4.55-4.70 (m, 1H, disappears with D₂O), 4.76-4.90 (m, 1H, CHOH), 5.61-5.69 (m, 2H, disappears with D₂O), 7.62 (s, 1H, H₃). IR (KBr): cm⁻¹ 3456, 3357, 3322 (NH, OH), 1693 (COOEt), 1665 (CONH).

(C₁₃H₂₂N₄O₄) C, H, N. analysed (% calculated / found) C: 52.34/52.44; H: 7.43/7.26; N: 18.78/18.92.

### EXAMPLE 3

### 1-(2-hydroxypropyl)-5-(3-benzylureido)-1H-pyrazole-4-carboxylic acid ethyl ester Ic

Yield 52%, melting point 72-73 °C. ¹H-NMR (CDCl₃): δ 1.20-1.35 (m, 6H, 2CH₃), 3.96-4.07 (m, 2H, CH₂N), 4.19 (q, J=7.0, 2H, CH₂O), 4.30 (d, J=5.8, 2H, CH₂Ar), 4.74-4.90 (m, 1H, CHOH), 5.10- 5.21 (m, 1H, disappears with D₂O), 5.41-5.76 (m, 2H, disappears with D₂O), 7.12-7.35 (m, 5H, Ar), 7.54 (s, 1H, H₃). IR (KBr): cm⁻¹ 3429, 3327, 3229 (NH, OH), 1703 (COOEt) 1688 (CONH).

(C₁₇H₂₂N₄O₄) C, H, N. analysed (% calculated / found) C: 58.95/59.04; H: 6.40/6.69; N: 16.17/15.95.

### EXAMPLE 4

### 1-(2-hydroxypropyl)-5-[3-(4-benzyl)piperazinylureido]-1H-pyrazole-4-carboxylic acid ethyl ester Id

Yield 58%, melting point 129-130 °C. ¹H-NMR (CDCl₃): δ 1.29-1.41 (m, 6H, 2CH₃), 2.34-2.50 (m, 4H, 2CH₂N piperaz.), 3.42-3.57 (m, 6H, 2CH₂N piperaz + CH₂Ar), 3.92-4.18 (m, 2H, CH₂N pyraz.), 4.28 (q, J=7.2, 2H, CH₂O), 4.73-4.88 (m, 1H, CHOH), 5.61-5.69 (m, 2H, disappears with D₂O), 7.23-7.36 (m, 5H, Ar), 7.60 (s, 1H, H₃). IR (KBr): cm⁻¹ 3402, 3302, 3225 (NH, OH), 1700-1680 (COOEt + CONH).

(C₂₁H₂₉N₅O₄) C, H, N. analysed (% calculated / found) C: 60.71/60.93; H: 7.04/7.26; N: 16.86/16.96.

### EXAMPLE 5

### 1-(2hydroxybutyl)-5-(3-isopropylureido)-1H-pyrazole-4-carboxylic acid ethyl ester Ii

Yield 51%, melting point 102-103 °C. ¹H-NMR (CDCl₃): δ 0.97 (t, J=7.4, 3H, CH₃), 1.19 (d, J=6.6, 6H, 2CH₃ isoprop.), 1.35 (t, J=7.0, 3H, CH₃CH₂O), 1.57-1.89 (m, 2H, CH₂CH), 3.75-3.93 (m, 1H, CHNH), 4.07-4.14 (m, 2H, CH₂N), 4.28 (q, J=7.0, 2H, CH₂O), 4.52-4.74 (m, 2H, CHOH + 1H which disappears with D₂O), 5.72-5.85 (m, 2H, disappears with D₂O), 7.60 (s, 1H, H3). IR (KBr): cm⁻¹ 3416, 3352, 3307 (NH, OH), 1700-1680 (COOEt + CONH).

(C₁₄H₂₄N₄O₄) C, H, N. analysed (% calculated / found) C: 53.83/53.86; H: 7.74/7.68; N: 17.94/17.93.

### EXAMPLE 6

### 1-(2-hydroxybutyl)-5-(3-benzylureido)-1H-pyrazole-4-carboxylic acid ethyl ester Ij

Yield 68%, melting point 99-101 °C. ¹H-NMR (CDCl₃): δ 0.89 (t, J=7.4, 3H, CH₃), 1.26 (t, J=7.2, 3H, CH₃CH₂O), 1.53-1.80 (m, 2H, CH₂CH), 4.00 (d, J=5.4, 2H, CH₂N), 4.14 (q, J=7.2, 2H, CH₂O), 4.30 (d, J=6.0, 2H, CH₂Ar), 4.53-4.66 (m, 1H, CH), 5.20- 5.30 (m, 1H, disappears with D₂O), 5.49-5.80 (m, 2H, disappears with D₂O), 7.14-7.33 (m, 5H, Ar), 7.56 (s, 1H, H₃). IR (KBr): cm-1 3424, 3298, 3320 (NH, OH), 1698 (COOEt), 1674 (CONH).

(C₁₈H₂₄N₄O₄) C, H, N. analysed (% calculated / found) C: 59.99/60.32; H: 6.71/6.67; N: 15.55/15.49.

### EXAMPLE 7

### 1-(2-hydroxybutyl)-5-[3-(4-benzyl)piperazinylureido)-1H-pyrazole-4-carboxylic acid ethyl ester Ik

Yield 47%, melting point 117-118 °C. ¹H-NMR (CDCl₃): δ 0.97 (t, J=7.4, 3H, CH₃), 1.35 (t, J=7.0, 3H, CH₃CH₂O), 1.60-1.92 (m, 2H, CH₂CH), 2.33-2.51 (m, 4H, 2CH₂N piperaz.), 3.43-3.60 (m, 6H, 2CH₂N piperaz + CH₂Ar), 4.02-4.10 (m, 2H, CH₂N pyraz.), 4.27 (q, J=7.0, 2H, CH₂O), 4.50-4.63 (m, 1H, CHOH), 5.78-5.92 (m, 2H, disappears with D₂O), 7.21-7.41 (m, 5H, Ar), 7.59 (s, 1H, H₃). IR (KBr): cm⁻¹ 3418, 3331, 3231 (NH, OH), 1700-1660 (COOEt + CONH).

(C₂₂H₃₁N₅O₄) C, H, N. analysed (% calculated / found) C: 61.52/61.58; H: 7.27/7.29; N: 16.31/16.12.

### EXAMPLE 8

### 1-(2-hydroxypentyl)-5-(3-isopropylureido)-1H-pyrazole-4-carboxylic acid ethyl ester Ip

Yield 49%, melting point 70-71 °C. ¹H-NMR (CDCl₃): δ 0.91 (t, J=7.2, 3H, CH₃), 1.19 (d, J=6.6, 6H, 2CH₃ isoprop.), 1.35 (t, J=7.2, 3H, CH₃CH₂O), 1.40-1.60 (m, 4H, 2CH₂), 3.73-3.90 (m, 1H, CHNH), 4.01-4.11 (m, 2H, CH₂N), 4.28 (q, J=7.2, 2H, CH₂O), 4.58-4.74 (m, 2H, CHOH + 1H which disappears with D₂O), 5.69-5.83 (m, 2H, disappears with D₂O), 7.60 (s, 1H, H₃). IR (KBr): cm⁻¹ 3420, 3350, 3312 (NH, OH), 1693-1680 (COOEt + CONH).

(C₁₅H₂₆N₄O₄) C, H, N. analysed (% calculated / found) C: 55.20/55.29; H: 8.03/8.17; N: 17.17/17.18.

### EXAMPLE 9

### 1-(2-hydroxypentyl)-5-(3-benzylureido)-1H-pyrazole-4-carboxylic acid ethyl ester Iq

Yield 50%, melting point 111-112 °C. ¹H-NMR (CDCl₃): δ 0.82 (t, J=7.2, 3H, CH₃), 1.26 (t, J=7.2, 3H, CH₃CH₂O), 1.31-1.69 (m, 4H, 2CH₂), 3.99 (d, J=5.4, 2H, CH₂N), 4.18 (q, J=7.2, 2H, CH₂O), 4.30 (d, J=5.8, 2H, CH₂Ar), 4.58-4.75 (m, 1H, CHOH), 5.18-5.29 (m, 1H, disappears with D₂O), 5.46-5.80 (m, 2H, disappears with D₂O), 7.13-7.34 (m, 5H, Ar ), 7.51 (s, 1H, H₃). IR (KBr): cm⁻¹ 3446, 3370, 3331 (NH, OH), 1724 (COOEt), 1694 (CONH).

(C₁₉H₂₆N₄O₄) C, H, N. analysed (% calculated / found) C: 60.95/60.87; H: 7.00/6.93; N: 14.96/15.09.

### EXAMPLE 10

### 1-(2-hydroxypentyl)-5-[3-(4-benzyl)piperazinylureido)-1H-pyrazole-4-carboxylic acid ethyl ester Ir

Yield 68%, melting point 128-129 °C. ¹H-NMR (CDCl₃): δ 0.91 (t, J=7.2, 3H, CH₃), 1.35 (t, J=7.2, 3H, CH₃CH₂O), 1.40-1.81 (m, 4H, 2CH₂), 2.37-2.51 (m, 4H, 2CH₂N piperaz.), 3.44-3.59 (m, 6H, 2CH₂N piperaz + CH₂Ar), 4.01-4.16 (m, 2H, CH₂N pyraz.), 4.28 (q, J=7.2, 2H, CH₂O), 4.55-4.70 (m, 1H, CHOH), 5.80-5.89 (m, 2H, disappears with D₂O), 7.27-7.41 (m, 5H, Ar), 7.60 (s, 1H, H₃). IR (KBr): cm⁻¹ 3402, 3307, 3227 (NH, OH), 1700-1680 (COOEt + CONH).

(C₂₃H₃₃N₅O₄) C, H, N. analysed (% calculated / found) C: 62.28/62.04; H: 7.50/7.34; N: 15.79/15.70.

### General procedure for 1-(2-hydroxyalkyl)-5-[3-(1-naphthyl)ureido]-1H-pyrazole-4-carboxylic acids ethyl esters Ih, Io

To a suspension of the suitable 5-amino-1-(2-hydroxyalkyl)-1*H-*pyrazole-4-carboxylic acid ethyl ester (2.2 mmoles) in anhydrous toluene (10 ml) α-naphthylisocyanate (0.51 g, 3 mmoles) was added and the reaction mixture was refluxed for 6 hours. After cooling, the white solids obtained were filtered and recrystallised from absolute ethanol.

### EXAMPLE 11

### 1-(2-hydroxypropyl)-5-[3-(1-naphthyl)ureido]-1H-pyrazole-4-carboxylic acid ethyl ester Ih

Yield 92%, melting point 124-126 °C. ¹H-NMR (CDCl₃): δ 1.26 (t, J=7.0, 3H, CH₃CH₂), 1.35 (d, J=6.4, 3H, CH₃CH), 3.90-4.10 (m, 2H, CH₂N), 4.18 (q, J=7.0, 2H, CH₂O), 4.86-5.08 (m, 1H, CHOH), 5.20-5.60 (m, 2H, disappears with D₂O), 6.96-7.92 (m, 9H, 7HAr + H₃ + 1H which disappears with D₂O). IR (KBr): cm⁻¹ 3414, 3322, 3224 (NH, OH), 1709 (COOEt), 1682 (CONH).

(C₂₀H₂₂N₄O₄) C, H, N. analysed (% calculated / found) C: 62.82/62.69; H: 5.80/5.96; N: 14.65/14.36.

### EXAMPLE 12

### 1-(2-hydroxybutyl)-5-[3-(1-naphthyl)ureido]-1H-pyrazole-4-carboxylic acid ethyl ester Io

Yield 60% melting point 121-123 °C. ¹H-NMR (DMSO-d₆): δ 0.88 (t, J=7.4, 3H, CH₃), 1.16 (t, J=7.2, 3H, CH₃CH₂O), 1.40-1.73 (m, 2H, CH₂CH), 3.98-4.18 (m, 4H, CH₂O + CH₂N), 4.90-5.05 (m, 1H, CHOH), 6.10-6.23 (m, 2H, disappears with D₂O), 7.28-7.93 (m, 8H, 7HAr + H₃), 9.34 (br s , 1H, disappears with D₂O). IR (KBr): cm⁻¹ 3450, 3394, 3323 (NH, OH), 1711 (COOEt), 1673 (CONH).

(C₂₁H₂₄N₄O₄) C, H, N. analysed (% calculated / found) C: 63.62/63.46; H: 6.10/5.98; N: 14.13/13.79.

### General procedure for 1-(2-hydroxyalkyl)-5-[3-(2/3/4-fluorophenyl)ureido]-1H-pyrazole-4-carboxylic acids ethyl esters Ie-g, Il-m

A mixture of the suitable 5-amino-1-(2-hydroxyalkyl)-1*H*-pyrazole-4-carboxylic acid ethyl ester (2.2 mmoles) and of the proper fluorophenylisocyanate (0.41 g, 3 mmoles) in anhydrous toluene (30 ml) was refluxed for 6 hours. After cooling, the solution was washed with HCl 3M (2x20 ml), with water (20 ml), dried (MgSO₄) and evaporated underreduced pressure. The crude crystallised by adding a solution of diethyl ether/petroleum ether (boiling point 50-60°C) 1/1. The white solids obtained were recrystallised from absolute ethanol.

### EXAMPLE 13

### 1-(2-hydroxypropyl)-5-[3-(2-fluorophenyl)ureido]-1H-pyrazole-4-carboxylic acid ethyl ester Ie

Yield 34%, melting point 131-132 °C. ¹H-NMR (CDCl₃): δ 1.27 (t, J = 7.0, 3H, CH₃CH₂), 1.35 (d, J=6.6, 3H, CH₃CH), 3.92-4.10 (m, 2H, CH₂N), 4.20 (q, J=7.0, 2H, CH₂O), 4.93-5.03 (m, 1H, CHOH), 5.36-5.55 (m, 2H, disappears with D₂O), 6.79-8.02 (m, 6H, 4HAr + H₃ +1H which disappears with D₂O). IR (KBr): cm⁻¹ 3425, 3335, 3235 (NH, OH), 1711 (COOEt), 1689 (CONH).

(C₁₆H₁₉N₄O₄F) C, H, N. analysed (% calculated / found) C: 54.85/54.66; H: 5.47/5.63; N: 15.99/15.93.

### EXAMPLE 14

### 1-(2-hydroxypropyl)-5-[3-(3-fluorophenyl)ureido]-1H-pyrazole-4-carboxylic acid ethyl ester If

Yield 48%, melting point 146-147 °C. ¹H-NMR (CDCl₃): δ 1.20-1.36 (m, 6H, 2CH₃), 3.90-4.07 (m, 2H, CH₂N), 4.20 (q, J=7.2, 2H, CH₂O), 4.84-5.03 (m, 1H, CHOH), 5.35-5.52 (m, 2H, disappears with D₂O), 6.65-7.30 (m, 5H, 4HAr + 1H which disappears with D₂O), 7.55 (s, 1H, H₃). IR (KBr): cm⁻¹ 3427, 3336, 3266 (NH, OH), 1712 (COOEt), 1689 (CONH).

(C₁₆H₁₉N₄O₄F) C, H, N. analysed (% calculated / found) C: 54.85/54.69; H: 5.47/5.50; N: 15.99/15.72.

### EXAMPLE 15

### 1-(2-hydroxypropyl)-5-[3-(4-fluorophenyl)ureido]-1H-pyrazole-4-carboxylic acid ethyl ester Ig

Yield 50%, melting point 175-177 °C. ¹H-NMR (CDCl₃): δ 1.14-1.36 (m, 6H, 2CH₃), 3.89-4.08 (m, 2H, CH₂N), 4.19 (q, J=7.2, 2H, CH₂O), 4.82-5.00 (m, 1H, CHOH), 5.36-5.54 (m, 2H, disappears with D₂O), 6.84-7.32 (m, 5H, 4HAr+1H which disappears with D₂O), 7.54 (s, 1H, H₃). IR (KBr): cm⁻¹ 3413, 3321, 3215 (NH, OH), 1709 (COOEt), 1682 (CONH).

(C₁₆H₁₉N₄O₄F) C, H, N. analysed (% calculated / found) C: 54.85/54.96; H: 5.47/5.37; N: 15.99/15.77.

### EXAMPLE 16

### 1-(2-hydroxybutyl)-5-[3-(2-fluorophenyl)ureido]-1H-pyrazole-4-carboxylic acid ethyl ester Il

Yield 42%, mp 99-100 °C. ¹H-NMR (CDCl₃): δ 0.95 (t, J=7.6, 3H, CH₃), 1.27 (t, J=7.0, 3H, CH₃CH₂O), 1.56-1.86 (m, 2H, CH₂CH), 4.07 (d, J=5.8, 2H, CH₂N), 4.20 (q, J=7.0, 2H, CH₂O), 4.63-4.80 (m, 1H, CHOH), 5.45-5.69 (m, 2H, disappears with D₂O), 6.82-8.06 (m, 6H, 4HAr + H₃ + 1H which disappears with D₂O). IR (KBr): cm⁻¹ 3460, 3350, 3211 (NH, OH), 1706 (COOEt), 1681 (CONH).

(C₁₇H₂₁N₄O₄F) C, H, N. analysed (% calculated / found) C: 56.04/55.99; H: 5.81/5.87; N: 15.38/15.48.

### EXAMPLE 17

### 1-(2-hydroxybutyl)-5-[3-(3-fluorophenyl)ureido]-1H-pyrazole-4-carboxylic acid ethyl ester Im

Yield 49%, melting point 128-129 °C. ¹H-NMR (CDCl₃): δ 0.91 (t, J=7.6, 3H, CH₃), 1.26 (t, J=7.2, 3H, CH₃CH₂O), 1.52-1.84 (m, 2H, CH₂CH), 4.03 (d, J=5.6, 2H, CH₂N), 4.19 (q, J=7.2, 2H, CH₂O), 4.63-4.77 (m, 1H, CHOH), 5.46-5.63 (m, 2H, disappears with D₂O), 6.64-7.38 (m, 5H, 4HAr + 1H which disappears with D₂O), 7.54 (s, 1H, H₃). IR (KBr): cm⁻¹ 3458, 3347, 3247 (NH, OH), 1707 (COOEt), 1680 (CONH).

(C₁₇H₂₁N₄O₄F) C, H, N. analysed (% calculated / found) C: 56.04/56.04; H: 5.81/5.67; N: 15.38/15.50.

### EXAMPLE 18

### 1-(2-hydroxybutyl)-5-[3-(4-fluorophenyl)ureido]-1H-pyrazole-4-carboxylic acid ethyl ester In

Yield 50%, melting point 130-131 °C. ¹H-NMR (CDCl₃): δ 0.93 (t, J=7.6, 3H, CH₃), 1.27 (t, J=7.2, 3H, CH₃CH₂O), 1.53-1.86 (m, 2H, CH₂CH), 4.06 (d, J=5.8, 2H, CH₂N), 4.19 (q, J=7.2, 2H, CH₂O), 4.59-4.76 (m, 1H, CHOH), 5.41-5.77 (m, 2H, disappears with D₂O), 6.80-7.41 (m, 5H, 4HAr + 1H which disappears with D₂O), 7.54 (s, 1H, H₃). IR (KBr): cm⁻¹ 3467, 3362, 3252 (NH, OH), 1710 (COOEt), 1682 (CONH).

(C₁₇H₂₁N₄O₄F) C, H, N. analysed (% calculated / found) C: 56.04/55.91; H: 5.81/6.08; N: 15.38/15.28.

### Synthesis of ethyl 5-amino-1-methyl-1H-pyrazole-4-carboxylate IIa

Compound IIa was synthesised following the method reported in Kopp, M.; Lancelot, J.C.; Dallenague, P.; Rault, S. Synthesis of Novel Pyrazolopyrrolopyrazines, Potential analogs of Sildenafil J. Het. Chem. 2001, 38, 1045-1050.

### General procedure for 5-amino-1-(2-hydroxyalkyl)-1H-pyrazole-4-carboxylic acids ethyl esters IIb,c,d

To a solution of the suitable hydrazine derivative R₁-NHNH₂ (20 mmoles) in anhydrous toluene (for compound IIb) or in absolute ethanol (for compounds IIc and IId) ethyl(ethoxymethylene)cyanoacetate (3.38 g, 20 mmoles) was added and the mixture was heated at 70-80°C for 8 hours. After cooling, the organic phase was washed with H₂O (20 ml), dried (MgSO₄) and concentrated under reduced pressure. The crude product was purified by flash chromatography using a mixture of CH₂Cl₂/CH₃OH (8:2) as eluent. The crude oils were crystallised by adding diethyl ether and the solids obtained were recrystallised from absolute ethanol.

### 5-amino-1-(2-hydroxypropyl)-1H-pyrazole-4-carboxplic acid ethyl ester IIb

Yield 61%, melting point 66-68 °C. ¹H-NMR (CDCl₃): δ 1.26 (d, J=6.4, 3H, CH₃CH), 1.35 (t, J=7.0, 3H, CH₃CH₂), 3.13 (br s, 1H, OH, disappears with D₂O), 3.78-4.42 (m, 5H, CH₂O + CH₂N + CH), 5.44 (br s, 2H, NH₂, disappears with D₂O), 7.61 (s, 1H, H₃). IR (KBr): cm⁻¹ 3426, 3390, 3313 (NH₂ + OH), 1697 (CO).

(C₉H₁₅N₃O₃) C, H, N. analysed (% calculated / found) C: 50.69/50.79; H: 7.09/7.06; N: 19.71/19.88.

### 5-amino-1-(2-hydroxybutyl)-1H-pyrazole-4-carboxylic acid ethyl ester IIc

Yield 61%, melting point 90-91 °C. ¹H-NMR (DMSO-d₆): δ 0.88 (t, J=7.4, 3H, CH₃), 1.23 (t, J=7.0, 3H, CH₃CH₂O), 1.24-1.43 (m, 2H, CH₂CH), 3.62-3.77 (m, 1H, CH), 3.85 (d, J=5.6, 2H, CH₂N), 4.16 (q, J=7.4, 2H, CH₂O), 4.98 (d, J = 5.2, 1H, OH, disappears with D₂O), 6.09 (br s, 2H, NH₂, disappears with D₂O), 7.45 (s, 1H, H₃). IR (KBr): cm⁻¹ 3433, 3343 (NH₂), 3209 (OH), 1689 (CO).

(C₁₀H₁₇N₃O₃) C, H, N. analysed (% calculated / found) C: 52.85/52.42; H: 7.54/7.60; N: 18.49/18.66.

### 5-amino-1-(2-hydroxypentyl)-1H-pyrazole-4-carboxylic acid ethyl ester IId

Yield 72%, melting point 84-86 °C. ¹H-NMR (CDCl₃): δ 0.95 (t, J=7.0, 3H, CH₃), 1.37 (t, J=7.0, 3H, CH₃CH₂O), 1.38-1.57 (m, 4H, 2CH₂), 3.19 (br s, 1H, OH, disappears with D₂O), 3.75-3.91 (m, 1H, CH), 3.86-4.08 (m, 2H, CH₂N), 4.25 (q, J=7.0, 2H, CH₂O), 5.46 (br s, 2H, NH₂, disappears with D₂O), 7.59 (s, 1H, H₃). IR (KBr): cm⁻¹ 3405, 3291, 3215 (NH₂ + OH), 1690 (CO).

(C₁₁H₁₉N₃O₃) C, H, N. analysed (% calculated / found) C: 54.76/54.74; H: 7.94/7.92; N: 17.41/17.44.

### Synthesis of 5-amino-1-(2-hydroxy-2-phenylethyl)-1H-pyrazole-4-carboxylic acid ethyl ester IIe

Compound IIe was synthesised following the method indicated in Schenone, S.; Bruno, O.; Fossa, P.; Ranise, A.; Menozzi, G.; Mosti, L.; Bondavalli, F.; Martini, C.; Trincavelli, L. Synthesis and biological data of 4-Amino-1-(2-chloro-2-phenylethyl)-1H-pyrazolo[3,4-b]pyridine-5-carboxylic acid ethyl esters, a new series of A1-Adenosine receptor (A1AR) ligands. Bioorg. Med. Chem. Lett. 2001, 11, 2529-2531.

### Synthesis of 1-hydrazino-propan-2-ol IIIb and of 1- hydrazinobutan-2-ol IIIc

Compounds IIIb and IIIc were synthesised following the method indicated in Gever, G. Hydrazinoalkanols JACS 1954, 76, 1283-1285.

### Synthesis of 1-hydrazinopentan-2-ol IIId

To hydrazine hydrate (30 ml, 0.6 mmoles) heated at 60-70°C 1,2-epoxypentane was added dropwise (14.64 g, 0.17 mmoles) and the mixture was heated at 90-95°C for 1 hour. Then, the excess of hydrazine hydrate was removed under reduced pressure and the crude was distilled bulb to bulb to yield a viscous oil, which becomes wax solid.

Yield 82%, boiling point 120°C/0.6 mmHg.

### Synthesis of 2-hydrazino-2-phenylethanol IIIe

Compound IIIe was synthesised following the method described in Benoit, G., Hydroxyalkyl hydrazines, Bull. Soc. Chi. Fr. 1939, 6, 708-715.

### Biological methods

The inhibitory activity of the compounds according to the present invention towards fMLP- or CXCL8-induced chemotaxiswas evaluated following methods described in the literature (Giordano, C. et al., Bioorg. Med. Chem. 2006, 14, 2642 and documents cited in such article).

A summary of such methods is given hereinafter.

### Preparation of the Neutrophils

Cells were obtained from blood of healthy subjects and human peripheral blood neutrophils were purified by using the standard techniques of dextran sedimentation (Pharmacia, Uppsala, Sweden), centrifugation on Ficoll-Paque (Pharmacia) and hypotonic lysis of contaminating red blood cells. The cells were washed twice and resuspended in Krebs-Ringer (KRPG) phosphate, pH 7.4, at a final concentration of 50 x 10⁶ cells/ml and kept at room temperature until used. The neutrophils were 98-100% viable, as determined using the Trypan blue exclusion test.

### Preparation of the chemoattractants and tested compounds

The f-MLP-OMe and the compounds to be tested (10-²M) were dissolved in dimethylsulfoxide (DMSO) while CXCL8 (10⁻⁵ M) was dissolved in water. All solutions were diluted in KRPG before use to obtain the final concentration required by the experimental protocol. At the concentrations used, the DMSO did not interfere with any of the biological assays carried out.

### Random Locomotion

A random locomotion test was carried out with a 48-well microchemotaxis chamber (Bio Probe, Milan) and migration into the filter was evaluated by the leading-front method (Gennari, C. et al, Eur. J. Org. Chem. (1998), p. 945) by estimating the distance in micrometres which the leading edge of the cell migrated. The actual control random movement is 35 ± 3 µm SE of six separate experiments carried out in duplicate.

### Chemotaxis

The directional movement or chemotaxis was measured with the same chamber used for the random locomotion, adding fMLP-OMe 10⁻⁹ M or CXCL8 10⁻⁸ M in the lower compartment.

Each compound to be tested was added in the upper compartment of the chemotaxis chamber, diluted at concentrations varying from 10⁻¹⁵ to 10⁻⁵ M with KRPG containing 1 mg/ml of bovine serum albumin (BSA; Orha Behringwerke, Germany).

The neutrophils were pre-incubated with the compounds to be tested ten minutes before the functional test. The data was expressed in terms of chemotactic index (C.I.), which is the ratio (migration towards test attractant - migration towards the buffer)/migration towards the buffer.

The C.I. of fMLP-OMe is 1.2 at 10⁻⁹ while the C.I. of CXCL8 is 1.02 at 10⁻⁸ M.

The antagonism was measured as C.I. in the absence (100%) and in the presence of the compound to be tested at different concentrations. CI₅₀ values were calculated from the sigmoid dose-response curve by a nonlinear regression analysis (Graph Pad Prism, San Diego, USA).

The values are means of six separate experiments carried out twice. The standard errors are in the range of 0.02-0.09 C.I.

The tests carried out on compounds according to the present invention provided the following results.

| Comp | R1 | Q | Inhibition of the migration of neutrophils induced by fMLP CI₅₀ (nM) | Inhibition of the migration of neutrophils induced by IL8 CI₅₀ (pM) |
|---|---|---|---|---|
| Ia | CH₂-CHOH-C₆H₅ | α-naphthylamino | 7.8 ± 0.52 | 7.60 ± 1.0 |
| Ib | CH₂-CHOH-CH₃ | isopropylamino | 90 ± 9.3 | 0.82 ± 0.07 |
| Ic | CH₂-CHOH-CH₃ | benzylamino | 15 ± 1.1 | 0.54 ± 0.06 |
| Id | CH₂-CHOH-CH₃ | N-benzylpiperazino | > 10 µM (63%) | 0.99 ± 0.08 |
| Ie | CH₂-CHOH-CH₃ | o-F-anilino | 100 ± 10.3 | 0.55 ± 0.01 |
| If | CH₂-CHOH-CH₃ | m-F-anilino | > 10 µM (60%) | 0.70 ± 0.01 |
| Ig | CH₂-CHOH-CH₃ | p-F-anilino | > 10 µM (55%) | 200 ± 60 |
| Ih | CH₂-CHOH-CH₃ | α-naphthylamino | 9.8 ± 0.90 | 0.97 ± 0.06 |
| Ii | CH₂-CHOH-C₂H₅ | isopropylamino | > 10 µM (52%) | 4.2 ± 0.7 |
| Ij | CH₂-CHOH-C₂H₅ | benzylamino | > 10 µM (54%) | 7.4 ± 0.2 |
| Ik | CH₂-CHOH-C₂H₅ | N-benzylpiperazino | 2000 ± 200 | 0.08 ± 0.003 |
| Il | CH₂-CHOH-C₂H₅ | o- F-anilino | 120 ± 12 | 1.6 ± 0.2 |
| Im | CH₂-CHOH-C₂H₅ | m- F-anilino | 0.19 ± 0.01 | 360 ± 60 |
| In | CH₂-CHOH-C₂H₅ | p- F-anilino | 3.2 ± 0.40 | 1900 ± 130 |
| Io | CH₂-CHOH-C₂H₅ | α-naphthylamino | 12 ± 1.2 | 9600 ± 1000 |
| Ip | CH₂-CHOH-C₃H₇ | isopropylamino | 20 ± 2.2 | 1000 ± 50 |
| Iq | CH₂-CHOH-C₃H₇ | benzylamino | 0.43 ± 0.05 | 3.2 ± 0.1 |
| Ir | CH₂-CHOH-C₃H₇ | N-benzylpiperazino | 1.1 ± 0.10 | 90 ± 4 |
| Is | CH₃ | isopropylamino | 77 ± 7.7 | 1.8 ± 0.5 |
| It | CH₃ | benzylamino | 18 ± 1.6 | 1300 ± 110 |
| Iu | CH₃ | N-benzylpiperazino | 150 ± 14.2 | 54.6 ± 0.5 |
| CSA | | | 0.047 ± 0.006 | |
| DMSO | | | > 10,000 | |

### Bibliographical References:

(1) Nauseef, W. et al., "Basic Principles in the Diagnosis and Management of Infectious Diseases", Mandel, Bennett, Dolin Eds.; Churchill Livingstone, NY, 2000, pp 89-112.
(2) Kobayashi, S. et al., "Regulation of the neutrophil mediated inflammatory response to infection", Microbes Infect., 2003, 5, 1337-1344.
(3) Delves, P. et al., The immune system. Second of two parts., Adv. Immunol., 2000.
(4) Katanaev, V. L., Signal transduction in neutrophil chemotaxis Biochemistry 2001, 66, 351-368.
(5) Panaro, M. A.; Mitolo, V. Cellular responses to FMLP challenging: A mini-review Immunopharmacol. Immunotoxicol. 1999, 21, 397- 419.
(6) Selvatici, R.; Falzarano, 5.; Mollica, A.; Spisani, S. Signal transduction pathways triggered by selective formylpeptide analogues in human neutrophils. Eur. J. Pharmacol. 2006, 534, 1-11.
(7) Hanahan, D. J. Platelet activating factor: A biologically active phosphoglyceride. Annu. Rev. Biochem. 1986, 55, 483-509.
(8) Crooks, 5. W.; Stockley, R. A. Leukotriene B4. Int. J. Biochem. Cell. Biol. 1998, 30, 173-178.
(9) Gerard, C.; Gerard, N. P. C5a anaphylatoxin and its seven transmembrane-segment receptor. Annu. Rev. Immunol.. 1994, 12, 775-808.
(10) Baggiolini, M. Chemokines and leukocyte traffic. Nature 1998, 392, 565-568.
(11) Hart, J.; Barish, G.; Murphy, P. M.; Gao, J. L., N-Formylpeptides induce two distinct concentration optima for mouse neutrophil chemotaxis by differential interaction with two N-formylpeptide receptor (FPR) subtypes: Molecular characterization of FPR2, a second mouse neutrophil FPR. J. Exp. Med. 1999, 190, 741-747.
(12) Le, Y.; Oppenheim, J. J.; Wang, J, M. Pleiotropic roles of formyl peptide receptors. Cytokine Growth Factor Rev. 2001, 12, 91-105.
(13) Holmes, W. E.; Lee, J.; Kuang, W. J.; Rice, G. C.; Wood, W. Structure and functional expression of a human interleukin-8 receptor. Science 1991, 253, 1278-1280.
(14) Murphy P, M.; Tiffany, H. L. Cloning of complementary DNA encoding a functional human interleukin-8 receptor. Science 1991, 253, 1280-1283.
(15) Zlotnik A.; Yoshie, O. Chemokines: A new classification system and their role in immunity. Immunity 2000, 12, 121-127.
(16) Chuntharapai, A.; Kim, J. K. Regulation of the expression of IL8 receptor A/B by IL8: Possible functions of each receptor. J. Immunol. 1995, 155, 2587-2594.
(17) Niggli, V. Signaling to migration in neutrophils: Importance of localized pathways. IJBCB 2003, 35, 1619-1638.
(18) Cicchetti, G.; Allen, P. G.; Glogauer, M. Chemotactic signaling pathways in neutrophils: From receptor to actin assembly. Crit. Rev. Oral Biol. Med. 2002, 13, 220-228.
(19) Stephens, L.; ElIson, C.; Hawkins, P. Roles of PI3Ks in leukocyte chemotaxis and phagocytosis. Curr. Opin. Cell. Biol. 2002, 14, 203-213.
(20) Weiner, O. D. Regulation of cell polarity during eukaryotic chemotaxis: The chemotactic compass. Curr. Opin. Cell. Biol. 2002, 14, 196-202.
(21) Fechheimer, M.; Zigmond, S. H. Changes in cytoskeletal proteins of polymorphonuclear leukocytes induced by chemotactic peptides. Cell. Motil. 1983, 3, 349-361.
(22) Valerius, N. E.; Stendahl, O.; Hartwig, 1. H.; Stossel, T. P. Distribution of actin-binding protein and myosin in polymorphonuclear leukocytes during locomotion and phagocytosis. Cell 1981, 24, 195-202.
(23) Wymann, M. P.; Kernen, P.; Bengtsson, T.; Andersson, T.; Baggiolini, M.; Deranleau, D. A. Corresponding oscillations in neutrophil shape and filamentous actin content..J. Biol. Chem. 1990, 265, 619-622.
(24) Condeelis, J. Life at the leading edge: The formation of cell protrusions. Annu, Rev. Cell. Biol. 1993, 9, 411-444.
(25) Elson, E. L.; Felder, S. F.; Jay, P. Y.; Kolodney, M. S.; Pasternak, C. Forces in cell locomotion. Biochem. Soc. Symp. 1999, 65, 299-314.
(26) Carter, S. B. Effects of cytochalasins on mammalian cells. Nature 1967, 213, 261-264.
(27) Becker, E. L.; Davis, A. T.; Estensen, R. D.; Quie, P. G. Cytochalasin B. IV. Inhibition and stimulation of chemotaxis of rabbit and human polymorphonuclear leukocytes. J. Immunol. 1972, 108, 396-402.
(28) Zigmond, S. H.; Hirsch, J. G. Effects of cytochalasin B on polymorphonuclear leukocyte locomotion, phagocytosis and glycolysis. Exp. Cell. Res. 1972, 73, 383-393.
(29) Norgauer, J.; Kownatzki, E.; Seifert, R.; Aktories, K. Botulinum C2 toxin ADP-ribosylates actin and enhances 02- production and secretion but inhibits migration of activated human neutrophils. J. Clin. Invest. 1988, 82, 1376-1382.
(30) Pollard, T. D.; Blanchoin, L.; Mullins, R. D. Molecular mechanisms controlling actin filament dynamics in nonmuscle cells. Annu. Rev. Biophys. Biomol. Struct. 2000, 29, 545-576.
(31) Li, Z.; Jiang, H.; Xie, W.; Zhang, Z.; Smrcka, A. V.; Wu, D. Roles of PLC-beta2 and -beta3 and Pl3Kgamma in chemoattractant-mediated signal transduction. Science 2000, 287, 1046-1049.
(32) Hirsch, E.; Katanaev, V. L.; Garlanda, C.; Azzolino, O.; Pirola, L.; Silengo, L.; Sozzani, S.; Mantovani, A.; Altruda, F.; Wymann, M. P. Central role for G protein-coupled phosphoinositide 3-kinase gamma in inflammation. Science 2000, 287, 1049-1053.
(33) Sadhu, C.; Masinovsky, B.; Dick, K.; Sowell, C. G.; Staunton, D. E. Essential role of phosphoinositide 3-kinase δ in neutrophil directional movement. J. Immunol. 2003, 170, 2647-2654.
(34) Wang, E.; Herzmark, P.; Weiner, O. D.; Srinivasan, S.; Servant, O.; Boume, H. R. Lipid products of PI(3)Ks maintain persistent cell polarity and directed motility in neutrophils. Nat. Cell. Biol. 2002, 4,513-518.
(35) Chodniewicz, D.; Zhelev, D. V. Novel pathways of F-actin polymerization in the human neutrophil. Blood 2003, 102, 2251-2258.
(36) Chodniewicz, D.; Zhelev, D. V. Chemoattractant receptor-stimulated F-actin polymerization in the human neutrophil is signaled by 2 distinct pathways. Blood 2003, 101, 1181-1184.
(37) Zhelev, D. V.; Alteraifi, A. M.; Chodniewicz D. Controlled pseudopod extension of human neutrophils stimulated with different chemoattractants. Biophys. J. 2004, 87, 688-695.
(38) White, J. R.; Lee, J. M.; Young, P. R.; Hertzberg, R. P.; Jurewicz, A. J.; Chaikin, M. A.; Widdowson, K. L.; Foley, J. J.; Martin, L. D.; Griswold, D. E.; Sarau, H. M. Identification of a potent, selective non-peptide CXCR2 antagonist that inhibits interleukin-8-induced neutrophil migration. J. Biol. Chem. 1998, 273, 10095-10098.
(39) Homk, E. Chemokine receptor antagonists. Med. Res. Rev. 2000, 20, 155-168.
(40) Johnson, Z. et al., Chemokine inhibition-Why, when, where, which and how? Biochem. Soc. Trans. 2004, 32, 366-377.
(41) Bruno, O., Brullo, C., Arduino, N., Schenone, S., Ranise, A., Bondavalli, F., Ottonello, L., Dapino, P., Dallegri, F. Synthesis and biological evaluation of neutrophilic inflammation inhibitors. Farmaco 2004, 59, 223-235.

## Claims

1. A compound of formula (I), 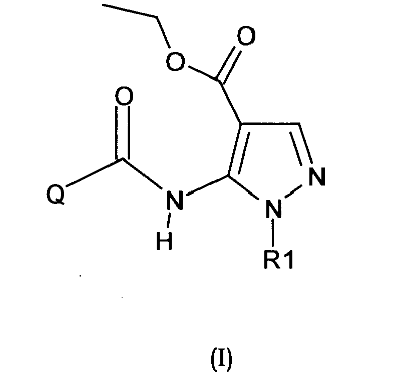 wherein:
R₁ = H, optionally substituted C₁-C₈ alkyl, -CH₂-CH(R₃)OR₂, where R₂ = H or C₁-C₃ alkyl and R₃ = optionally substituted C₁-C₈ alkyl;
Q = NHR₄ or a saturated heterocyclic 3-7 membered ring containing nitrogen, wherein R₄ = optionally substituted C₃-C₈ alkyl, optionally substituted aralkyl, optionally substituted aryl;
provided that, when R₁ = H or CH₃, Q is different from anilino and chloroanilino,
and enantiomers, diastereoisomers and pharmaceutically acceptable salts thereof.

2. A compound according to claim 1, wherein
R₁ = C₁-C₅ alkyl, -CH₂-CH(R₃)OR₂, wherein R₃ = C₁-C₃ alkyl and R₂ is defined as in claim 1;
Q = -NH-CH(CH₃)₂, -NH-CH₂-C₆H₅, -NH-C₆H₄X, N-benzylpiperazino, cyclopropylamino, pyrrolidino, piperidino, morpholino, N-methylpiperazino or α-naphthylamino, wherein X = halogen.

3. A compound according to claim 2, wherein
Q = -NH-CH(CH₃)₂, -NH-CH₂-C₆H₅, -NH-C₆H₄X, N-benzylpiperazino or α-naphthylamino, where X = halogen.

4. A compound according to claim 2, wherein
R₁ = -CH₂-CH(OH)CH₃, -CH₂-CH(OH)C₂H₅, -CH₂-CH(OH)C₃H₇ and
Q = -NH-CH(CH₃)₂, -NH-CH₂-C₆H₅, N-benzylpiperazino, α-naphthylamino, o-fluoroanilino or m-fluoroanilino.

5. A compound according to claim 4, having the following formula:
- 1-(2-hydroxypropyl)-5-(3-isopropylureido)-1H-pyrazole-4-carboxylic acid ethyl ester;
- 1-(2-hydroxy-2-phenylethyl)-5-[3-(1-naphthyl)ureido]-1H-pyrazole-4-carboxylic acid ethyl ester;
- 1-(2-hydroxypropyl)-5-(3-benzylureido)-1H-pyrazole-4-carboxylic acid ethyl ester;
- 1-(2-hydroxypropyl)-5-[3-(4-benzyl)piperazinylureido]-1H-pyrazole-4-carboxylic acid ethyl ester;
- 1-(2-hydroxybutyl)-5-(3-isopropylureido)-1H-pyrazole-4-carboxylic acid ethyl ester;
- 1-(2-hydroxybutyl)-5-(3-benzylureido)-1H-pyrazole-4-carboxylic acid ethyl ester;
- 1-(2-hydroxybutyl)-5-[3-(4-benzyl)piperazinylureido)-1H-pyrazole-4-carboxylic acid ethyl ester;
- 1-(2-hydroxypentyl)-5-(3-isopropylureido)-1H-pyrazole-4-carboxylic acid ethyl ester;
- 1-(2-hydroxypentyl)-5-(3-benzylureido)-1H-pyrazole-4-carboxylic acid ethyl ester;
- 1-(2-hydroxypentyl)-5-[3-(4-benzyl)piperazinylureido)-1H-pyrazole-4-carboxylic acid ethyl ester;
- 1-(2-hydroxypropyl)-5-[3-(1-naphthyl)ureido]-1H-pyrazole-4-carboxylic acid ethyl ester;
- 1-(2-hydroxybutyl)-5-[3-(1-naphthyl)ureido]-1H-pyrazole-4-carboxylic acid ethyl ester;
- 1-(2-hydroxypropyl)-5-[3-(2-fluorophenyl)ureido]-1H-pyrazole-4-carboxylic acid ethyl ester;
- 1-(2-hydroxypropyl)-5-[3-(3-fluorophenyl)ureido]-1H-pyrazole-4-carboxylic acid ethyl ester;
- 1-(2-hydroxypropyl)-5-[3-(4-fluorophenyl)ureido]-1H-pyrazole-4-carboxylic acid ethyl ester;
- 1-(2-hydroxybutyl)-5-[3-(2-fluorophenyl)ureido]-1H-pyrazole-4-carboxylic acid ethyl ester;
- 1-(2-hydroxybutyl)-5-[3-(3-fluorophenyl)ureido]-1H-pyrazole-4-carboxylic acid ethyl ester;
- 1-(2-hydroxybutyl)-5-[3-(4-fluorophenyl)ureido]-1H-pyrazole-4-carboxylic acid ethyl ester.

6. A compound of formula (I) 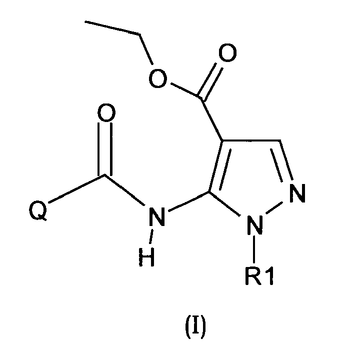 wherein:
R₁ = H, optionally substituted C₁-C₈ alkyl, -CH₂-CH(R₃)OR₂, where R₂ = H or C₁-C₃ alkyl and R₃ = optionally substituted C₁-C₈ alkyl;
Q = NHR₄ or a saturated heterocyclic 3-7 membered ring containing nitrogen, where R₄ = optionally substituted C₃-C₈ alkyl, optionally substituted aralkyl, optionally substituted aryl,
and enantiomers, diastereoisomers and pharmaceutically acceptable salts thereof for use as a medicament with anti-inflammatory activity.

7. A compound according to claim 6, wherein:
R₁ = C₁-C₅ alkyl, -CH₂-CH(R₃)OR₂, where R₃ = C₁-C₃ alkyl and R₂ is defined as in claim 5;
Q = -NH-CH(CH₃)₂, -NH-CH₂-C₆H₅, -NH-C₆H₄X, N-benzylpiperazino, cyclopropylamino, pyrrolidino, piperidino, morpholino, N-methylpiperazino or α-naphthylamino, where X = halogen,
and enantiomers, diastereoisomers and pharmaceutically acceptable salts thereof for use as a medicament with anti-inflammatory activity.

8. A compound according to claim 7, wherein:
Q = -NH-CH(CH₃)₂, -NH-CH₂-C₆H₅, -NH-C₆H₄X, N-benzylpiperazino or α-naphthylamino, where X = halogen, and enantiomers, diastereoisomers and pharmaceutically acceptable salts thereof for use as a medicament with anti-inflammatory activity.

9. A compound according to claim 8, wherein
R₁ = -CH₂-CH(OH)CH₃, -CH₂-CH(OH)C₂H₅, -CH₂-CH(OH)C₃H₇ and
Q = -NH-CH(CH₃)₂, -NH-CH₂-C₆H₅, N-benzylpiperazino, α-naphthylamino, o-fluoroanilino or m-fluoroanilino,
and enantiomers, diastereoisomers and pharmaceutically acceptable salts thereof for use as a medicament with anti-inflammatory activity.

10. A compound according to any one of claims 1 to 5 and enantiomers, diastereoisomers and pharmaceutically acceptable salts thereof for use as a medicament.

11. A compound according to any one of claims 1 to 5 and enantiomers, diastereoisomers and pharmaceutically acceptable salts thereof for use as an agent which inhibits neutrophil chemotaxis induced by interleukin 8.

12. A compound according to any one of claims 6 to 9 for use in the treatment of inflammatory pathologies of autoimmune nature.

13. Use of a compound according to any one of claims 1 to 9 for the manufacture of a medicament for the treatment of inflammatory pathologies, in particular of inflammatory pathologies of autoimmune nature.

14. Use of a compound according to any one of claims 1 to 9 for the manufacture of a medicament for the treatment of psoriasis, ulcerative colitis, glomerulonephritis, acute respiratory failure, cystic fibrosis, rheumatoid arthritis, chronic obstructive lung disease.

15. Use of a compound according to any one of claims 1 to 9 for the manufacture of a medicament for the prevention and treatment of damages from ischemia-reperfusion.

16. A pharmaceutical composition comprising at least one compound according to any one of claims 1 to 5 and a pharmaceutically acceptable carrier.

17. A process for the preparation of a compound according to any one of claims 1 to 5, comprising the reaction of a compound of formula (II) 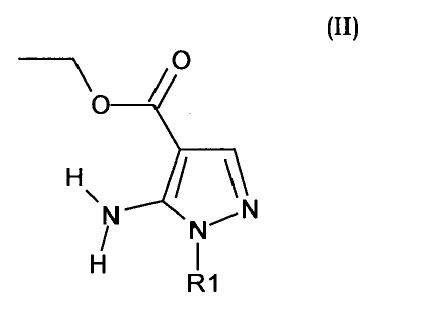 wherein R₁ is as defined in claim 1, with COCl₂ and subsequently with an amine of formula QH, where Q is defined as in claim 1.

18. A process for the preparation of a compound according to any one of claims 1 to 5, comprising the reaction of a compound of formula (II) 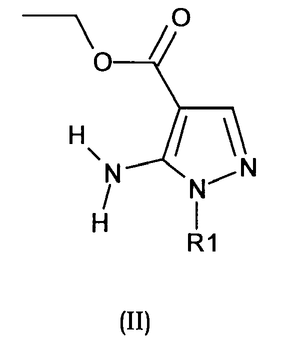 wherein R₁ is as defined in claim 1, with an isocyanate of formula QNCO, where Q represents an optionally substituted aryl.
